# EUROPEAN PATENT APPLICATION

(11) **EP 0 576 754 A1**
(43) Date of publication of application: **05.01.1994**
(21) Application number: 92306138.6
(22) Date of filing: 02.07.1992
(51) Int. Cl.: A61F 5/445

(54) **Device for use by ostomy patients**

(71) Applicant: Knox, Patrick Robert William, Abbeylands Isle of Man (GB); Holt, David Arthur, Douglas Isle of Man (GB); Dryden, Harry Peter, Baldrine Isle of Man (GB)
(72) Inventor: Knox, Patrick Robert William, Abbeylands Isle of Man (GB); Holt, David Arthur, Douglas Isle of Man (GB); Dryden, Harry Peter, Baldrine Isle of Man (GB)
(74) Representative: Jones, William

(57) **Abstract**

A waste matter disposal device for use by ostomy patients comprises a frame (1) and a chute (11) depending therefrom, the frame being adapted to rest on the lap of a seated patient with the chute hanging between the thighs of the patient, and with the chute so connected to the frame that in its normally intended attitude of use as just outlined, an opening is defined within the periphery of the frame to allow the contents of the user's ostomy pouch to be deposited into the chute. The lower (in use) end of the chute is closed, and the lower end region is formed in an inherently flexible sheet, the whole arrangement thereby allowing the chute and its contents to be formed into a sealed bag by the user and disposed of directly.

## Description

### Field of the Invention

This invention relates to a device for use by ostomy patients, in particular patients who have undergone ileosostomy or urostomy surgery.

### Background to the Invention

The evacuation and disposal of contents of ostomy pouch is an unpleasant chore which has to be undertaken on a daily basis by ostomy patients. A basic practise is to unfasten the pouch and liberate its contents into the basin of a toilet. It is least uncomfortable for the patient to carry out the procedure when seated. However, a problem arises in effectively directing the effluent from the pouch into the toilet.

A number of devices have been developed to assist the ostomy patient in emptying his/her pouch. Two such devices are described in a United Kingdom Patent application, No 2606274A, and United Kingdom Patent No 2216779, both filed in the name of the present applicant. The later patent describes a device comprising a frame work adapted to be placed upon the lap of a patient seated upon a toilet, the frame having a chute depending from it and extending between the legs of the patient into the basin of the toilet to allow the contents of an ostomy pouch to be deposited in and flow through the chute. In the described embodiments having an open end the pouch effluent drains from the lower end of the chute into the bowl of the toilet using the user to retract the chute and collapse it into a compact state for disposal. Throughout this later stage fluid may continue to leak from the outlet of the chute.

It is a general objective of the present invention to provide a device which will yet further improve the cleanliness of the pouch emptying procedure.

It is a more specific object of the present invention to improve upon the device described and illustrated in United Kingdom (GB) patent specification No 2 216 779 which currently forms the closest prior art known to the applicant.

### Summary of the Invention

In its broadest aspect, the present invention provides a waste matter disposal device for use by ostomy patients, the device comprising a frame adapted to rest, in use, in a generally stable manner on the lap of a seated patient; and a chute adapted to hang, in use, from the frame so as to extend between the legs - usually between the thighs - of the patient; and with means connecting the chute to the frame so that, with the device in its intended attitude of use as outlined above, an opening is defined within the periphery of the frame to allow the contents of the user's ostomy pouch to be deposited into the chute; characterised by the feature that the lower (in-use) end of the chute is closed so that the chute will function, in use, as a receptacle for the pouch contents; and by the further feature - known in itself - that the material of at least the lower, waste-matter-containing, region of the chute is an inherently flexible sheet; and these characterising features thereby allowing the chute and its contents to be formed into a sealed bag by the user and disposed of directly.

With such an arrangement there is no need for a separate disposal bag and no risk of spilling the contents of the chute; or indeed of contaminating anything else with the soiled chute even though the chute contents may have been successfully disposed of. All these problems are avoided by this very effective specific modification to the construction specifically described and illustrated in Figure 10 of the previous specification (GB) 2 216 779.

Advantageously the disposable part of the device is composed of a degradable material, i.e a material which will disintergrate over a period of time and within a relatively finite time limit, preferably one which will disintergrate to a substantial extent with a period of at most three days and more preferably still within a relatively immediate time period of, for example, between 30 minutes and 1 hour. In that last-named instance there is then no significant risk of drain blockage by the filled bag if it is deposited into and flushed from a conventional toilet bowl.

Such a degradable material may have a high bio-degradable content and, in a presently preferred embodiment of the invention (to be described and illustrated) it comprises a water-soluble plastics sheet which is preferably hot-water-soluble; or which at least tends automatically to heat-seal with or without pressure being applied to it.

The frame itself may be a relatively permanent structure and one such is illustrated in specification (GB) 2 216 779 where the frame is described as being of a "plug-in" construction. In accordance with another advantageous development of the present invention, the frame comprises a dissembleable "snap-fit" assembly of frame members. Given the inherently flexible and collapsible nature of at least part of the chute, the ability to dissemble the frame enables the frame and the chute to be sold as a "kit of parts" package; and it also enables the frame to be relatively easily brought back into usage if part of it - for whatever reason - is contaminated or damaged, by simply replacing the affected member with a new one.

The means connecting the chute to the frame in use may comprise projections spaced about the frame and interacting with holes corresponding spaced about the open upper end region of the chute, again as illustrated previously in specification (GB) 2 216 779. But the present invention extends this concept by forming hole-periphery-defining lines of weakness - e.g perforations - in the material around the open upper end region of the chute, so that the holes are actually formed at the time of usage by the user pushing-out the chute material within those peripheral lines. This could cut down the manufacturing and/or assembly costs of the frame-and-chute kit.

### Brief Description of Drawings

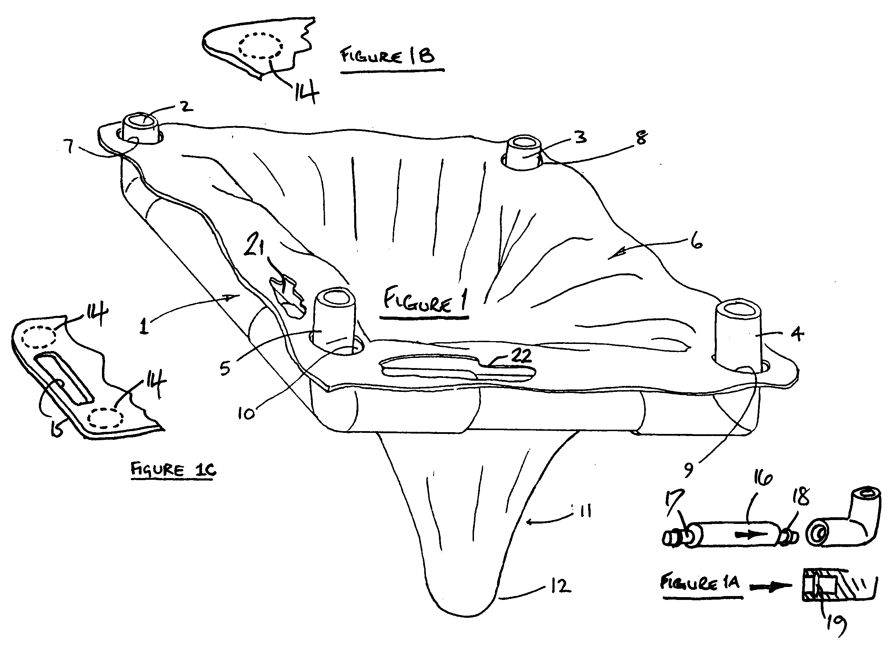

Figure 1 of the accompanying drawings is a perspective view of a preferred embodiment of the present invention, whilst Figures 1A, 1B and 1C respectively are part-views illustrating modifications to the main Figure 1 embodiment.

### Description of the Preferred Embodiment

As shown in Figure 1, the device comprises a rectangular frame 1 formed from "plug-in" rigid moulded plastics fittings, the frame having upstanding corner pieces 2 through 5, the device also comprising a shaped flexible plastics sheet 6 formed with corner holes 7 through 10 whose spacing is identical to the relative spacing of the upstanding posts 2 through 5. The sheet 6 is so sized and shaped that when the holes 7 through 10 are fitted over their respective corner posts 2 through 5, the sheet 6 forms a chute 11 extending through the central opening of the frame 1 such as to pass downwardly between the legs or thighs of the patient on whose lap the frame is rested in use. The chute 11 has a closed lower, in use, end 12 such as to form a receptacle for the contents of the patients ostomy pouch. The upper, in use, portion of the sheet 6 which is fastened to the frame acts as a basin to direct the emptied contents of the pouch into the basin 12 of the chute 11.

The plastics material of which the sheet 6 forming the chute 11 is composed suitably comprises a hot water soluble plastics material such as, for example, poly vinyl alcohol.

A convenient procedure for use of the device will now be described. Firstly, the patient sits upon the seat of a toilet and places the frame 1 onto his/her lap with the corner posts 2 through 5 extending upwardly. The patient fits the sheet 6 over the frame 1 as described above so that the chute 11 formed by the sheet 6 extends down between the patients legs into the toilet bowl.

The patient then un-clips his/her ostomy pouch and gently manipulates the pouch over the basin formed by the sheet 6 to release the pouch and pour its contents into the chute 11. That done, the patient then cleans and re-clips the ostomy pouch and proceeds to lift the sheet 6 from fitment to the frame 1 holding the corners of the sheet 6 apart and gently lowering the receptacle formed by the sheet 6 into the water within the toilet bowl.

As the chute 11 sinks into the water the material thereof is compressed around the waste matter from the ostomy pouch and expels air from the chute 11 sealing the mouth of the chute 11 above the waste matter by adhesion of the water soluble plastics material as it closes above the waste matter and is partially dissolved by the water and the heat of the waste matter.

By avoiding drawing the corners of the sheet 6 together prior to entry of the chute 11 into the water the air is expelled uniformly from the chute 11 so that no air is trapped therewithin and consequently the bag formed by the chute 11 will sink unimpeded.

The bag enclosing the waste material formed of the chute 11 will continue to disintegrate over a period of generally between 30 minutes and 1 hour so that there is no significant risk of drain blockage by the bag as it is flushed from the toilet.

By closing the lower end of the chute 11 and disposing of it within the toilet there is no need to withdraw the chute 11 from the toilet bowl and spillage is avoided.

Although the present invention has been described above with respect to one preferred embodiment numerous alternative embodiments are conceivable within the scope of the invention. The construction of the frame 1 and the manner of fitment of the chute 11 to the frame 1 may be varied to suit a range of cost, compactness and comfort criteria. The frame 1 may, for example, be rebated to conform to the stomach of the user as described in UK Patent No 2216779. The sheet 6 need not have pre-formed holes 7 through 10. These holes may be pierced in the material when required.

Figure 1C shows such an embodiment in which circular lines of weakness - in this instance, perforations - 14 are formed at each opposite corner of the sheet 6 (i.e one such circular line at each of the four opposite corners, although only two of course are illustrated in Figure 1C). This same Figure also shows a finger-receiving cut out 15 in the sheet - which could also be defined by a line of weakness and left for the user to push out at the point of usage - to make the sheet as a whole rather more easy to grip securely when transferring the sheet from the frame into the toilet bowl after usage. Again, there are two such cut outs 15, one on each opposite side of the sheet periphery.

In Figure 1B the circular hole-periphery-defining array is an array of perforations, rather than just a series of weakened areas formed in the sheet material. And in Figure 1A, finally, the "snap-in" nature of the frame members is illustrated; circular-cylindrical plastics frame member 16 ending, at each of its opposite ends, in a reduced-diameter section 17 which exhibits a raised integrally-moulded collar 18 and in use each such collar 18 snaps into and out of a recess 19 moulded into the next frame member.

For the avoidance of doubt, the supposed cut outs 21 and 22 shown in the sheet 6 in Figure 1 are, of course, portions deliberately cut away (ie. by way of artistic licence) in order to show the form of the frame members beneath the sheet . They are not, literally, cut out of the sheet 6.

## Claims

1. A waste matter disposal device for use by ostomy patients, the device comprising a frame adapted to rest, in use, in a generally stable manner on the lap of a seated patient; and a chute adapted to hang, in use, from the frame so as to extend between the legs - usually between the thighs - of the patient; and with means connecting the chute to the frame so that, with the device in its intended attitude of use as outlined above, an opening is defined within the periphery of the frame to allow the contents of the user's ostomy pouch to be deposited into the chute; characterised by the feature that the lower (in-use) end of the chute is closed so that the chute will function, in use, as a receptacle for the pouch contents; and by the further feature - known in itself - that the material of at least the lower, waste-matter-containing, region of the chute is an inherently flexible sheet; and these characterising features thereby allowing the chute and its contents to be formed into a sealed bag by the user and disposed of directly.

2. A device according to Claim 1 and characterised by the feature that the disposable part of the device is composed of a degradable material, preferably one which will disintegrate within a period of 3 days, more preferably within a period of between 30 minutes and 1 hour.

3. A device according to Claim 2 and characterised by the feature that the waster-matter-containing region of the chute comprises a water-soluble plastics sheet, preferably one which tends automatically to heat-seal.

4. A device according to any of Claims 1 to 3 and characterised by the feature that the frame comprises a dissembleable "snap-fit" assembly of frame members.

5. A device according to any of Claims 1 to 4 and characterised in that the means connecting the chute to the frame in use comprise projections spaced about the frame and interacting with holes correspondingly spaced about the open upper end region of the chute; and further characterised by the presence of hole-periphery-defining lines of weakness - for example, perforations - in the chute material so that the holes are formed by the user pushing-out the chute material within those lines at the point of usage.
